# EUROPEAN PATENT SPECIFICATION

(11) **EP 1 604 211 B1**
(45) Date of publication and mention of the grant of the patent: **30.04.2008**
(21) Application number: 04717679.7
(22) Date of filing: 05.03.2004
(51) Int. Cl.: G01N 33/94, G01N 33/566

(54) **IDENTIFICATION OF THERAPEUTIC COMPOUNDS**
IDENTIFIKATION VON THERAPEUTISCHE VERBINDUNGEN.
IDENTIFICATION DE COMPOSES THERAPEUTIQUES

(30) Priority: 07.03.2003 GB 0305153
(43) Date of publication of application: 14.12.2005
(73) Proprietor: Cambridge Biotechnology Ltd, Cambridge CB2 1XJ (GB)
(72) Inventor: RICHARDSON, Peter, Hadstock, Cambridge CB1 6PF (GB)
(74) Representative: Höglund, Lars
(86) International application number: PCT/GB2004/000902
(87) International publication number: WO 2004/079329

(56) References cited:
- WO-A1-20/04052377
- FR-A- 2 162 128
- US-A- 5 506 213
- US-A- 5 877 180
- SAWYNOK JANA: "Adenosine receptor activation and nociception" EUROPEAN JOURNAL OF PHARMACOLOGY, vol. 347, no. 1, 17 April 1998 (1998-04-17), pages 1-11, XP002287446 ISSN: 0014-2999
- BARTLETT R T ET AL: "Synthesis and pharmacological evaluation of a series of analogues of 1-methylisoguanosine." JOURNAL OF MEDICINAL CHEMISTRY. AUG 1981, vol. 24, no. 8, August 1981 (1981-08), pages 947-954, XP002409379 ISSN: 0022-2623
- PAN H L ET AL: "Allosteric adenosine modulation to reduce allodynia." ANESTHESIOLOGY. AUG 2001, vol. 95, no. 2, August 2001 (2001-08), pages 416-420, XP008072083 ISSN: 0003-3022
- RANE K ET AL: "Intrathecal adenosine administration: a phase 1 clinical safety study in healthy volunteers, with additional evaluation of its influence on sensory thresholds and experimental pain." ANESTHESIOLOGY. NOV 1998, vol. 89, no. 5, November 1998 (1998-11), pages 1108-1115 ; di, XP008072110 ISSN: 0003-3022

## Description

This invention relates to the identification of potential therapeutic agents.

In normal mammalian tissues extracellular pH is tightly regulated between pH 7.35 and 7.45. Some tissues experience lower pH values, particularly the lumen of the stomach (pH between 2 and 3) and the surfaces of some epithelia (for example, the lung surface pH is approximately 6.8, Jayaraman, S. et al. (2001) Am. J. Physiol. Cell Physiol. 281, C1504-1511).

In pathological tissues, for example during inflammation, ischaemia and other types of damage, a reduction in pH occurs. For instance, in the arthritic joint, reductions in pH of up to 1 unit are typically seen (Andersson, S. E. et al. (1999) J. Rheumatol. 26, 2018-2024). It has recently been shown that cartilage in osteoarthritic joints experiences pH values as low as 5.0 (Konttinnen, Y. T. et al., (2002) Arth. Rheum. 46, 953-960). Excessive neuronal activity can also result in a reduction in extracellular pH. For instance, epileptic discharges and overstimulation of the spinal cord have been associated with changes in extracellular pH (Chesler, M. Prog. Neurobiol. (1990) 34, 401-427). Similarly, reductions in extracellular pH have been observed in solid tumours where pH values as low as 5.5 have been observed, probably as consequence of poor perfusion and the predominance of glycolytic metabolism (Thistlethwaite, A. J. et al. Radiation Oncology Biol. Phys. (1985) 11, 1647-1652). In ischaemia the lack of blood supply results in a fall in pH (Iminke, D. C. & McCleskey, E. W. (2001) Nature Neurosci. 4, 869-870) and subsequent inflammatory cell activation.

A common factor in all these diseases and conditions is that the energy demands of the tissues involved outstrip the supply. This results in anaerobic metabolism with the production of lactic and pyruvic acids. Another contributory factor to pH changes is the emptying of secretory vacuoles (for example in nerve endings or inflammatory cells) into the extracellular space, since the contents of these vacuoles are maintained at low pH. The reduction in pH can help to protect the tissue. For example, the NMDA receptor in the CNS, which has been strongly implicated in ischaemic damage, is inhibited at pH 6.8.

Another common factor in the above diseases is adenosine. Adenosine is a ubiquitous local hormone/neurotransmitter that acts on four known receptors, the adenosine A1, A2A, A2B and A3 receptors. Adenosine generally serves to balance the supply and demand of energy in tissues. For example, in the heart released adenosine slows the heart by an A1 receptor mediated action in the nodes and atria (Belardinelli, L & Isenberg, G Am. J. Physiol. 224, H734-H737), while simultaneously dilating the coronary artery to increase energy (i.e. glucose, fat and oxygen) supply (Knabb et al., Circ. Res. (1983) 53, 33-41). Similarly during inflammation adenosine serves to inhibit inflammatory activity, while in conditions of excessive nerve activity activity (e.g. epilepsy) adenosine inhibits nerve firing (Klitgaard et al., Eur J. Pharmacol. (1993) 242, 221-228). This system, or a variant on it, is present in all tissues. Thus, under conditions where the pH falls, local adenosine serves to balance the energy supply and demand thus restoring normal tissue function and pH.

Adenosine itself can be used to diagnose and treat supraventricular tachycardia. Adenosine A1 receptor agonists are known to act as powerful analgesics (Sawynok, Eur J Pharmacol. (1998) 347, 1-11). Adenosine A2A receptor agonists are known to act as anti-inflammatory agents (for example, from US 5,877,180 and WO 99/34804). In experimental animals, A2A receptor agonists have been shown to be effective against a wide variety of conditions including sepsis, arthritis, and ischaemia/reperfusion injury arising from renal, coronary or cerebral artery occlusion. The common factor in these conditions is a reduction in the inflammatory response caused by the inhibitory effect of this receptor on most, if not all, inflammatory cells.

However, the ubiquitous distribution of adenosine receptors means that administration of adenosine receptor agonists causes adverse side effects. This has generally precluded the development of adenosine-based therapies. Selective A1 receptor agonists cause bradycardia. The first selective A2A receptor agonist (2-[4-(2-carboxyethyl)phenylethylamino]-5'-N-ethylcarboxamidoadenosine, or CGS21680), was tested in a Phase 2A clinical trial as a potential anti-hypertensive. However, administration caused a large fall in blood pressure and increase in cardiac output.

FR2162128 discloses that adenosine derivatives (including 2-alkoxy adenosine derivatives comprising a lower alkyl group of not less than two carbon atoms) have hypotensive and coronary vasodilatory activity.

Bartlett et al (J. Med. Chem. 1981, 24, 947-954) discloses the evaluation of analogues of 1-methylisoguanosine. These analogues include 2-methoxyadenosine (also known as spongosine). This and other compounds were tested for their skeletal muscle-relaxant, hypothermic, cardiovascular and anti-inflammatory effects in rodents following oral administration. 2-methoxyadenosine caused 25% inhibition of carageenan-induced inflammation in rats at 20 mg/kg po. However, reductions in mean blood pressure (41%), and heart rate (25%) were also observed after administration of this compound at this dose.

Kirk and Richardson (British Journal of Pharmacology (1995) 114, 537-543) describe characterization of [3H]-CGS 21680 binding sites in the rat striatum and cortex.

WO 96/38728 discloses a method for measuring the A2a receptor binding activity of compounds of pharmacological interest by the use of the tritiated ligand (³H)-SCH 58261.

Jiang et al. (J. Med. Chem. 1997, 40, 2588-2595) describe use of site-directed mutagenesis in the transmembrane helical domains of the human A_{2A} adenosine receptor to study structure-activity relationships for ligand binding.

Ribeiro et al. (Progress in Neurobiology 68 (2003) 377-392) is a review of adenosine receptors in the nervous system, and comments that peripheral side effects associated with adenosine receptor agonists limit their usefulness in therapeutics.

There is, therefore, a need to provide adenosine receptor agonists that can be administered with minimal side effects.

Askalan and Richardson (J. Neurochem. (1994) 63:1477-1484), describes the role of histidine residues in the adenosine A2A receptor ligand-binding site. In particular, the pH-dependency of the binding of ligands was examined. A two- to four-fold increase in affinity was observed for some ligands on lowering the ambient pH from 7.0 to 5.5. However, the action of adenosine A2A receptors was not studied.

Hiley et al (British Journal of Pharmacology (1995) 116, 2641-2646) investigated the effects of pH on responses to adenosine in the isolated perfused superior mesenteric arterial bed of the rat. Reducing the pH of the perfusate to 6.8 enhanced the dilator responses to adenosine by 10-fold.

The applicant has now surprisingly found that, at relatively low pH values within the physiological range, some compounds have substantially higher (approximately 100-fold higher) affinity for and/or efficacy of action at adenosine receptors than at higher pH values. It is believed that this is because the receptors change their conformation in response to changes in pH.

The applicant has appreciated that the pH sensitivity of adenosine receptors can be exploited to identify other adenosine receptor ligands that only have high receptor affinity and/or efficacy under conditions of low pH. The actions of such adenosine receptor ligands may then be targeted to regions of low pH, such as pathological tissues, without causing the serious side effects associated with administration of known adenosine receptor ligands.

According to the invention there is provided a method for identifying a potential therapeutic agent, which comprises determining the affinity and/or efficacy of a test compound for an adenosine receptor at a higher pH of at least pH 7.4, and also at a lower pH, from 5.5 to 7.2, or from 5.0 to 7.0, and identifying the compound as a said agent if the affinity and/or efficacy at the lower pH is greater than that at the higher pH.

It is preferred that the difference between the affinity of the potential therapeutic agent for, and/or its efficacy of action at, the adenosine receptor at the lower and higher pH is as great as possible. Where there is a large difference in affinity and/or efficacy, it is expected that the agent can be administered at a dosage at which it has beneficial therapeutic effects, and any side effects associated with higher doses of the agent are avoided or minimised. Preferably the affinity and/or efficacy at the lower pH is over 10 times, more preferably over 100 times, most preferably over 1000 times, greater than the affinity and/or efficacy at the higher pH.

Agonists that have higher affinity and/or efficacy at lower pH may be useful in therapy, for example in the prevention, treatment, or amelioration of pain, particularly neuropathic or chronic inflammatory pain and arthritis. The selectivity of these agonists means that they can be targeted to pathological tissues, for example to arthritic joints, thereby allowing effective administration of lower doses than suggested by the prior art, and minimising side effects.

Methods of the invention may be used to identify ligands that only bind to the adenosine A2A receptor with high affinity at low pH, when the receptor is believed to adopt a different conformation from that existing at pH 7.4. As these ligands are only effective agonists at low pH, they may only act at sites within the body where pH is depressed, most notably in the joint capsule of arthritis and at similar inflammatory pain sites.

Methods of the invention may be used to identify ligands that only bind to the adenosine A1 receptor with high affinity at low pH. Such ligands are expected to be useful in reducing excessive tissue activity, for example nervous activity, and to have reduced side effects compared to known adenosine A1 receptor agonists. Side effects of the known agonists include bradycardia and arteritis.

Methods of the invention may be used to identify ligands that only bind to the adenosine A3 receptor with high affinity at low pH. Such ligands are expected to be useful in the treatment of inflammatory disorders.

Based on the information provided herein, one of ordinary skill in the art can identify suitable active agents for the treatment of specific disease states. These agents would be selectively active in the pathological tissue, thus reducing the probability and severity of side effects associated with receptors in normal tissue.

In isolated tissue and cell preparations, pH sensitivity can be measured accurately. Therefore, after evidence has been obtained in animal models of pathology (for example, inflammatory pain), compounds meeting the appropriate criterion will serve as "hits" for subsequent drug discovery.

Compounds identified using methods of the invention are expected to be of use in the therapy, including prophylaxis, of various conditions. These include diseases or conditions in which prevention, treatment, or amelioration of the disease or condition is mediated by activation of adenosine receptors. Examples include diseases or conditions in which local tissue energy demand exceeds supply and/or pH falls, such as inflammation, ischemia-reperfusion injury, excessive neuronal activity (for example in epilepsy, or chronic pain or hyperalgesia, including inflammatory and neuropathic pain), sepsis, septic shock, neurodegeneration (for example Alzheimer's disease), and other conditions where energy demand exceeds supply, for example muscle fatigue or athletes' cramp.

Compounds identified using methods of the invention may be effective in the prevention, treatment, or amelioration of pain, in particular the following types of pain: bowel pain, pancreatic pain, pelvic/perineal pain, back pain, lower back pain, chest pain, cardiac pain, pelvic pain/PID, joint pain (for example, associated with tendonitis, bursitis, acute arthritis), neck pain, obstetric pain (labour or Caesarean-Section), cancer pain, HIV pain, phantom limb pain, post-operative pain, chronic neuropathic pain, failed back surgery pain, post physical trauma pain (including pain caused by a gunshot wound, a road traffic accident, or a burn), scar tissue pain, acute herpes Zoster pain, acute pancreatitis breakthrough pain (cancer), post-herpes neuralgia, trigeminal neuralgia.

Compounds identified using methods of the invention may be effective in the prevention, treatment, or amelioration of neuropathic or other pain caused by, or associated with diabetic neuropathy, polyneuropathy, fibromyalgia, myofascial pain syndrome, osteoarthritis, rheumatoid arthritis, sciatica or lumbar radiculopathy, spinal stenosis, temporo-mandibular joint disorder, renal colic, dysmenorrhoea/endometriosis.

Compounds identified using methods of the invention may be effective in the prevention, treatment, or amelioration of inflammatory or other pain caused by, or associated with arthritic conditions such as osteoarthritis, rheumatoid arthritis, rheumatoid spondylitis, gouty arthritis, or asthma, chronic obstructive pulmonary disease, fibrosis, multiple sclerosis, sepsis, septic shock, endotoxic shock, gram negative shock, toxic shock, hemorrhagic shock, adult respiratory distress syndrome, cerebral malaria, organ transplant rejection, pain secondary to cancer, HIV, chronic pulmonary inflammatory disease, silicosis, pulmonary sarcosis, bone resorption diseases, reperfusion injury, graft v. host rejection, multiple sclerosis, myasthenia gravis, allograft rejections, fever and myalgia due to infection, AIDS related complex (ARC), keloid formation, scar tissue formation, Crohn's disease, ulcerative colitis and pyresis, irritable bowel syndrome, osteoporosis, cerebral malaria, bacterial meningitis, or adverse effects from amphotericin B treatment, interleukin-2 treatment, OKT3 treatment, or GM-CSF treatment.

Compounds identified by a method of the invention may be particularly effective for the prevention, treatment, or amelioration of particular types of inflammation, including arthritis (particularly at the joint capsule of arthritis), asthma, psoriasis, and bowel inflammation.

Compounds identified by a method of the invention may be particularly effective in the prevention, treatment, or amelioration of rheumatoid arthritis, irritable bowel syndrome or osteoarthritis.

The utility of a compound may be determined by a radioligand assay described below. The increase in affinity can be measured for the high affinity binding site (i.e. the agonist active site), while the efficacy of the agonist at different pH values can be determined by assessing the difference in the affinity of the low and high affinity states. This can be done using GTP and stable analogues thereof, which convert the high affinity state of the receptor to the low affinity state thus mimicking activation of the receptor by agonists. Ligands with no efficacy (i.e. antagonists) do not differentiate between these different affinity states. Alternatively, agonists that have different affinities and/or efficacies at different pH values can be identified in functional assays, such as measurement of second messenger production or removal (e.g. stimulation or inhibition of cAMP accumulation, inositol phosphate turnover, calcium signalling, kinase activation etc).

The affinity of a test compound for an adenosine receptor may be determined by any of the following methods:
i) binding of multiple concentrations of labelled test compound (including radiolabelled test compound) to adenosine receptors present in tissues, tissue slices, intact cells, disrupted cells or cell derived membranes;
ii) displacement of a bound labelled compound from adenosine receptors by incubation with unlabelled test compound using tissues, tissue slices, intact cells, disrupted cells or cell derived membranes bearing such receptors. The labelled compound may be either the test compound, or a selective ligand for the receptor.

It will be appreciated that the efficacy of an agonist reflects its ability to activate its receptor. Agonists with high efficacy elicit a maximum receptor response, and partial agonists elicit a smaller response. The efficacy of action of a test compound at an adenosine receptor may be determined by any of the following methods:
i) determining adenosine receptor action by the measurement of the accumulation or depletion of signalling molecules including cAMP, IP3 and free calcium in tissues, tissue slices, intact cells or partially disrupted cells;
ii) use of biological membranes to assess adenosine receptor activation in response to a test compound by the measurement of G protein activation (for example by the use of radiolabelled guanine nuceotides), or by the measurement of enzyme activity (for example adenylyl cyclase, phosphodiesterase, phospholipase or protein kinase), or by the measurement of ion flow through ion channels activated by the adenosine receptor (for example calcium or potassium channels);
iii) determining adenosine receptor action by the measurement of protein kinase activity in tissues, tissue slices, intact cells, disrupted cells or cell derived membranes;
iv) determining adenosine receptor action by the measurement of phospholipase activity (e.g. phospholipase C, phospholipase A2, phospholipase D) in tissues, tissue slices, intact cells, disrupted cells or cell derived membranes;
v) determining adenosine receptor action by the measurement of protein phosphorylation and dephosphorylation in tissues, tissue slices, intact cells, disrupted cells or cell derived membranes.

The level of activity of a compound (i.e. its intrinsic efficacy) can vary depending on the effect desired. For instance, low efficacy agonists may be useful in reducing the probability of receptor desensitisation, or in conferring an extra dimension of targeting, i.e. to those tissues with large receptor reserve. The activity is preferably at least 50%, more preferably at least as great as that for any of the active agents reported below. The difference is preferably seen between physiological pH (7.4) and pH 5.5 or above, e.g. 6.2 (extreme for ischaemic tissue), 6.5, 7.0 (typical in areas of chronic inflammation) or 7.2. Alternatively, low efficacy partial agonists can be used as functional antagonists, binding to the receptor and preventing the binding of the endogenous transmitter/hormone (for example, adenosine).

If a potential therapeutic agent is identified, it may then be determined whether the agent has a therapeutic effect, for example against pain or inflammation (particularly any of the diseases or conditions listed above). Preferably a non human animal model is used to determine whether the identified agent has a therapeutic effect. Any suitable animal model may be used. Examples include arthritis induced by injection of collagen II, or neuropathic pain induced by streptozotocin induced diabetes.

Preferably the therapeutic effect of the identified agent is determined at a concentration below the EC50 value of the agent at the adenosine receptor at pH 7.4. More preferably the therapeutic effect is determined at a concentration that is one ten thousandth to one fifth, or one ten thousandth to one twentieth, or one ten thousandth to one hundredth, most preferably one ten thousandth to one thousandth, of the EC50 value.

To confirm that the identified agent may be administered with minimised side effects, the side effects (such as bradycardia, hypotension or tachycardia) caused by the identified agent may be determined. Preferably these are determined at a concentration below the EC50 value of the agent at the adenosine receptor at pH 7.4. More preferably the side effects are determined at a concentration that is one ten thousandth to one fifth, or one ten thousandth to one twentieth, or one ten thousandth to one hundredth, most preferably one ten thousandth to one thousandth, of the EC50 value.

A number of compounds that are agonists of the adenosine A1 or A2 receptor have been identified. These compounds have higher affinity for the adenosine A1 or A2 receptor at lower pH. It is believed that the receptors change their conformation in response to the changes in pH. The precise mechanism by which this change occurs is unknown, but it is thought to involve histidine residues which have been implicated in agonist binding to these receptors and which have pK values in the physiological range.

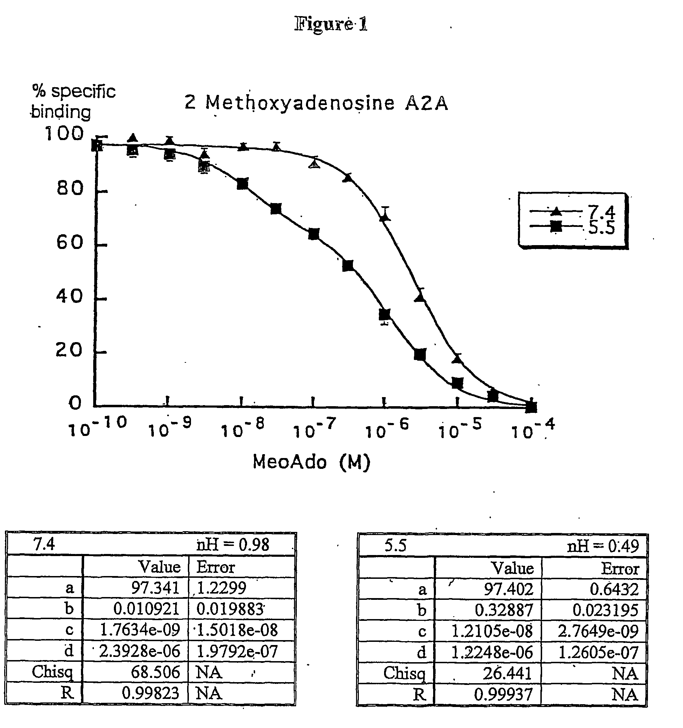

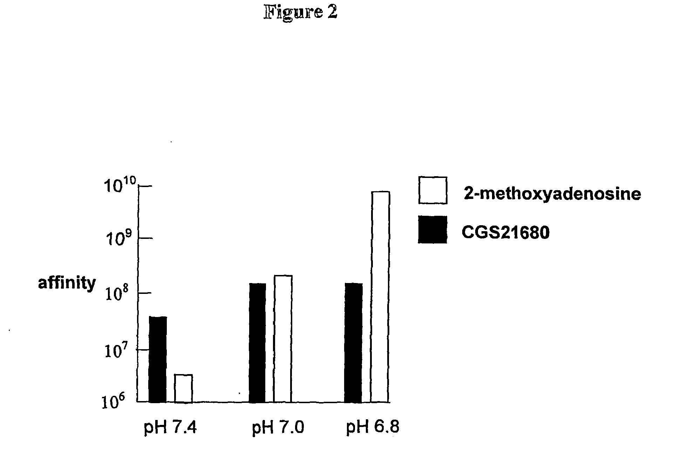

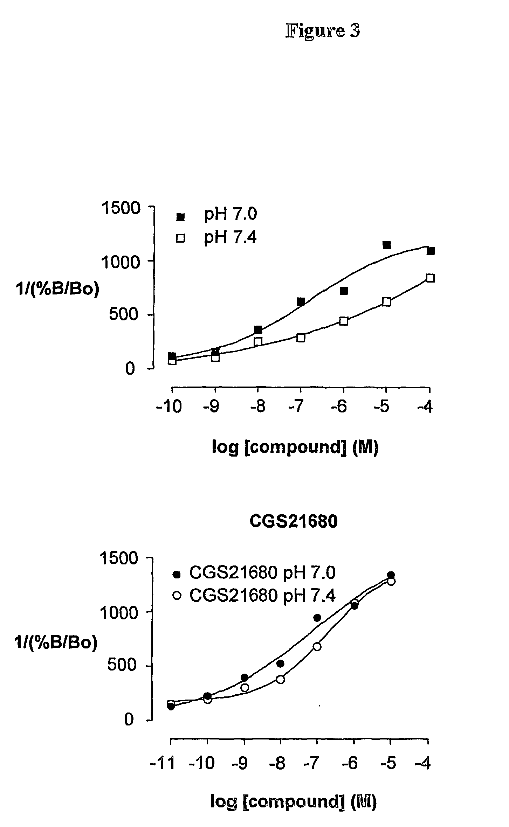

Figure 1 illustrates the increase in affinity observed with 2-methoxyadenosine as the pH in a ligand binding experiment was reduced from pH 7.4 to pH 5.5. Figure 2 demonstrates that the same increase in affinity was observed at pH7.0 and pH6.8, but that the archetypal A2A receptor agonist CGS21680 does not show this effect. Another surprising consequence of the affinity changes was that the efficacy of 2-methoxyadenosine at the adenosine A2A receptor was increased approximately 100 fold (Figure 3). In contrast 3'-deoxy-2-methoxyadenosine showed a decrease in affinity at the A1 receptor when the pH was lowered from 7.4 to 6.2. The Ki values determined for the high affinity sites in the displacement of [3H]-DPCPX from the human A1 receptor were 158 ± 85 nM at pH7.4 and 405 ± 114 nM at pH6.2. Thus, it appears that adenosine receptor agonists and partial agonists can be profoundly affected by local changes in tissue pH and, surprisingly, that the efficacy of these ligands can be either increased or decreased. Compounds that show a decrease in efficacy are likely to act as low efficacy partial agonists.

The applicant has appreciated that potential therapeutic agents may also be identified by administering a test compound *in vivo* or *in vitro* at a dose lower than that expected to activate (at pH 7.4) a significant proportion of adenosine receptors (i.e. sufficient adenosine receptors to elicit a beneficial therapeutic effect), and then assessing the difference in the dose required to activate the receptors in the pathological tissue compared to normal tissue.

According to a further aspect of the invention there is provided a method for identifying a potential therapeutic agent which comprises contacting a test compound with an adenosine receptor (an A1, A2A, A2B, or A3 adenosine receptor) in a pathological tissue, cell, or membrane and a corresponding normal tissue, cell, or membrane at a concentration below the EC50 value of the test compound at the adenosine receptor at pH 7.4, and determining whether there is any difference in action of the adenosine receptor in response to contact with the test compound between the normal tissue and the pathological tissue.

Preferably the test compound is contacted with the adenosine receptor at a concentration that is one ten thousandth to one fifth, or one ten thousandth to one twentieth, or one ten thousandth to one hundredth, most preferably one ten thousandth to one thousandth, of the EC50 value.

The test compound is identified as a potential therapeutic agent if the test compound is an agonist of the adenosine receptor, and the action of the adenosine receptor in response to the test compound is greater in the pathological tissue than the normal tissue. If the pathological tissue comprises epithelial tissue, potential therapeutic compounds for the treatment of epithelial disease (for example psoriasis, asthma, COPD) may be identified.

Adenosine receptor agonists that have different receptor affinity and/or efficacy at different pH, or which cause different action of adenosine receptors in pathological tissue compared to normal tissue, include derivatives of adenosine. Thus, it will be appreciated that methods of the invention may be used as screening methods, in particular to screen derivatives of adenosine. Derivatives that have low affinity (i.e. a Kd for an adenosine receptor >0.5µM) and/or efficacy at pH7.4 are preferred. The selectivity of the derivatives for different adenosine receptors may change at reduced pH, accordingly the selectivity of a given compound should be determined at both normal and reduced pH.

Compounds of the following general formulae not being part of the present invention have been found, many of which are believed to have a higher affinity for adenosine receptors, and/or efficacy of action at adenosine receptors, at lower pH: 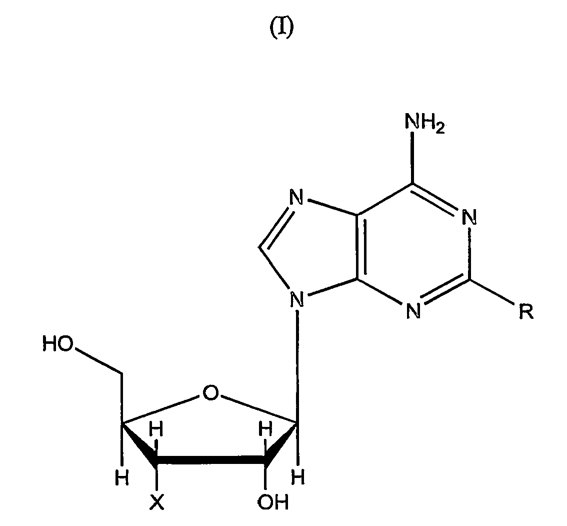 wherein R is C₁₋₄ alkoxy and X is OH; 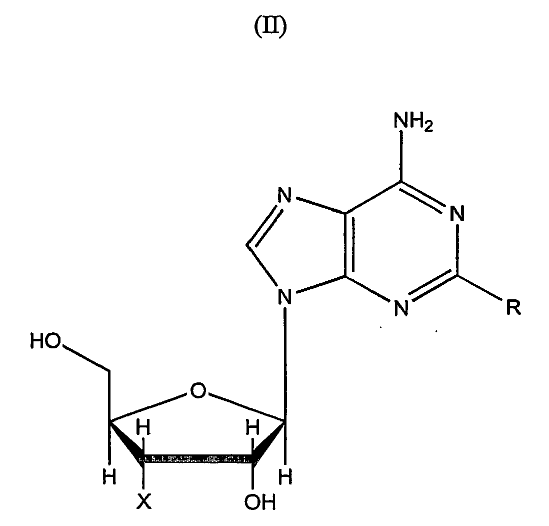 wherein R is C₁₋₄ alkoxy, and X is H.

Compounds exemplified in Example 10 have been found to have a higher affinity for adenosine A2A receptors at lower pH.

Compounds of general formula (I) or (II), or Example 10, that have a higher affinity for adenosine receptors, and/or efficacy of action at adenosine receptors, at lower pH may be used as medicaments in the prevention, treatment, or amelioration of various diseases or conditions. These include diseases or conditions in which prevention, treatment, or amelioration is mediated by activation of adenosine receptors. Examples include diseases or conditions in which local tissue energy demand exceeds supply and/or pH falls.

Compounds of general formula (I) or (II), or Example 10, that have a higher affinity for adenosine receptors, and/or efficacy of action at adenosine receptors, at lower pH may be used in particular for the manufacture of a medicament for the prevention, treatment, or amelioration of cancer, inflammation, ischemia-reperfusion injury, pain, excessive neuronal activity (for example in epilepsy, or chronic pain or hyperalgesia, including inflammatory and neuropathic pain), sepsis, septic shock, neurodegeneration (including Alzheimer's Disease), muscle fatigue or muscle cramp (particularly athletes' cramp).

Compounds of general formula (I) or (II), or Example 10, that have a higher affinity for adenosine receptors, and/or efficacy of action at adenosine receptors, at lower pH may be effective in the prevention, treatment, or amelioration of the following types of pain: bowel pain, pancreatic pain, pelvic/perineal pain, back pain, lower back pain, chest pain, cardiac pain, pelvic pain/PID, joint pain (for example, associated with tendonitis, bursitis, acute arthritis), neck pain, obstetric pain (labour or Caesarean-Section), cancer pain, HIV pain, phantom limb pain, post-operative pain, chronic neuropathic pain, failed back surgery pain, post physical trauma pain (including pain caused by a gunshot wound, a road traffic accident, or a burn), scar tissue pain, acute herpes Zoster pain, acute pancreatitis breakthrough pain (cancer), post-herpes neuralgia, trigeminal neuralgia; neuropathic or other pain caused by, or associated with diabetic neuropathy, polyneuropathy, fibromyalgia, myofascial pain syndrome, osteoarthritis, rheumatoid arthritis, sciatica or lumbar radiculopathy, spinal stenosis, temporo-mandibular joint disorder, renal colic, dysmenorrhoea/endometriosis; or inflammatory or other pain caused by, or associated with arthritic conditions such as osteoarthritis, rheumatoid arthritis, rheumatoid spondylitis, gouty arthritis, or asthma, chronic obstructive pulmonary disease, fibrosis, multiple sclerosis, sepsis, septic shock, endotoxic shock, gram negative shock, toxic shock, hemorrhagic shock, adult respiratory distress syndrome, cerebral malaria, organ transplant rejection, pain secondary to cancer, HIV, chronic pulmonary inflammatory disease, silicosis, pulmonary sarcosis, bone resorption diseases, reperfusion injury, graft v. host rejection, multiple sclerosis, myasthenia gravis, allograft rejections, fever and myalgia due to infection, AIDS related complex (ARC), keloid formation, scar tissue formation, Crohn's disease, ulcerative colitis and pyresis, irritable bowel syndrome, osteoporosis, cerebral malaria, bacterial meningitis, or adverse effects from amphotericin B treatment, interleukin-2 treatment, OKT3 treatment, or GM-CSF treatment.

Compounds of formula (I), compounds of formula (II), and compounds of Example 10, that have a higher affinity for adenosine receptors, and/or efficacy of action at adenosine receptors, at lower pH may be particularly effective for the prevention, treatment, or amelioration of particular types of inflammation, including arthritis (particularly at the joint capsule of arthritis), asthma, psoriasis, and bowel inflammation.

Compounds of formula (I), compounds of formula (II), and compounds of Example 10, that have a higher affinity for adenosine receptors, and/or efficacy of action at adenosine receptors, at lower pH may be particularly effective in the prevention, treatment, or amelioration of rheumatoid arthritis, irritable bowel syndrome or osteoarthritis.

Compounds of formula (I) include: 2-methoxyadenosine, 2-ethoxyadenosine, 2-propoxyadenosine, 2-isopropoxyadenosine, and 2-butoxyadenosine. Preferred compounds of formula (I) are 2-methoxyadenosine, 2-ethoxyadenosine, and 2-butyloxyadenosine.

Compounds of formula (II) include: 3'-deoxy-2-methoxyadenosine, 3'-deoxy-2-ethoxyadenosiiie, 3'-deoxy-2-propoxyadenosine, 3'-deoxy-2-isopropoxyadenosine, and 3'-deoxy-2-butoxyadenosine. Preferred compounds of formula (ll) are 3'-deoxy-2-propoxyadenosine, 3'-deoxy-2-isopropoxyadenosine, and 3'-deoxy-2-butoxyadenosine.

2-methoxyadenosine has been reported to have an EC50 value at the adenosine A2A receptor of 3 µM (Daly, J.W. et al. (1993) Pharmacol. 46, 91-100). However, this compound surprisingly has profound anti-hyperalgesic and anti-inflammatory activity at plasma concentrations of 0.2µM or less. At these low doses 2-methoxyadenosine has reduced probability and severity of side effects. The activity of 2-methoxyadenosine as an analgesic is the subject of International patent application no. PCT/GB03/05379 (unpublished at the filing date of the present application).

Other compounds of formula (I) and compounds of formula (II) (and compounds of Example 10) that have a higher affinity for adenosine receptors, and/or efficacy of action at adenosine receptors, at lower pH (and compounds identified using methods of the invention) are also believed to be much more effective at low doses than other adenosine receptor agonists. Thus, it is expected that such compounds can be effectively administered at doses at which they have reduced probability and severity of side effects. These compounds may alternatively or additionally have reduced probability and severity of side effects compared to other adenosine receptor agonists.

Pain has two components, each involving activation of sensory neurons. The first component is the early or immediate phase when a sensory neuron is stimulated, for instance as the result of heat or pressure on the skin. The second component is the consequence of an increased sensitivity of the sensory mechanisms innervating tissue which has been previously damaged. This second component is referred to as hyperlagesia, and is involved in all forms of chronic pain arising from tissue damage, but not in the early or immediate phase of pain perception.

Thus, hyperalgesia is a condition of heightened pain perception caused by tissue damage. This condition is a natural response of the nervous system apparently designed to encourage protection of the damaged tissue by an injured individual, to give time for tissue repair to occur. There are two known underlying causes of this condition, an increase in sensory neuron activity, and a change in neuronal processing of nociceptive information which occurs in the spinal cord. Hyperalgesia can be debilitating in conditions of chronic inflammation (e.g. rheumatoid arthritis), and when sensory nerve damage has occurred (i.e. neuropathic pain).

Compounds identified by methods of the invention, compounds of formula (I) or (II), and compounds of Example 10, that have a higher affinity for adenosine receptors, and/or efficacy of action at adenosine receptors, at lower pH are believed to be effective in inhibiting pain perception in mammals suffering from neuropathic and inflammatory pain even when administered at doses expected to give concentrations well below those known to activate adenosine receptors. At these doses it is believed that these compounds can treat neuropathic and inflammatory pain without causing the significant side effects associated with administration of other adenosine receptor agonists, and also without reducing normal sensory perception.

Compounds identified by methods of the invention, compounds of formula (I) or (II), of compounds of Example 10, that have a higher affinity for adenosine receptors, and/or efficacy of action at adenosine receptors, at lower pH can be used as anti-hyperalgesics for the prevention, treatment, or amelioration of pain (particularly hyperalgesia) caused as a result of neuropathy, including bowel pain, back pain, cancer pain, HIV pain, phantom limb pain, post-operative pain, diabetic neuropathy, polyneuropathy, post-herpes neuralgia, and trigeminal neuralgia.

Compounds identified by methods of the invention, compounds of formula (I) or (II), or compounds of Example 10, that have a higher affinity for adenosine receptors, and/or efficacy of action at adenosine receptors, at lower pH can be used as anti-hyperalgesics for the prevention, treatment, or amelioration of pain (particularly hyperalgesia) caused as a result of inflammatory disease, including bowel pain, back pain, cancer pain, fibromyalgia, post-operative pain, osteoarthritis, and rheumatoid arthritis.

Compounds identified by methods of the invention, compounds of formula (I) or (II), and compounds of Example 10, that have a higher affinity for adenosine receptors, and/or efficacy of action at adenosine receptors, at lower pH are expected to have advantages (such as increased efficacy and/or reduced side effects) when used to treat pain (particularly hyperalgesia) compared to compounds of the two major classes of known analgesics. These are: (i) non steroidal anti-inflammatory drugs (NSAIDs) and the related COX-2 inhibitors; and (ii) opiates based on morphine. Analgesics of both these classes are effective in controlling normal nociceptive pain. However, they are less effective against some types of hyperalgesic pain, such as neuropathic pain. Many medical practitioners are reluctant to prescribe opiates at the high doses required to affect neuropathic pain because of the side effects caused by administration of these compounds, and the possibility that patients may become addicted to them. NSAIDs are much less potent than opiates, so even higher doses of these compounds are required. However, this is undesirable because these compounds cause irritation of the gastro-intestinal tract.

The amount of a compound identified by a method of the invention, or of formula (I) or (II), or of Example 10, that is administered to a subject should be an amount which gives rise to a peak plasma concentration that is less than the EC50 value of the compound at adenosine receptors at pH 7.4.

It will be appreciated that the EC50 value of the compound is likely to be different for different adenosine receptors (i.e. the A1, A2A, A2B, A3 adenosine receptors). The amount of the compound that is to be administered should be calculated relative to the lowest EC50 value of the compound at the different receptors.

The peak plasma concentration may be one thousandth to one fifth, or one fiftieth to one third (more preferably one thousandth to one twentieth, one hundredth or one fiftieth to one fifth, one fiftieth to one tenth, or one tenth to one fifth) of the EC50 value. Preferably the peak plasma concentration is one ten thousandth to one fifth, or one ten thousandth to one twentieth, or one ten thousandth to one hundredth, most preferably one ten thousandth to one thousandth of the EC50 value.

Preferably the amount administered gives rise to a plasma concentration that is maintained for more than one hour between one thousandth and one fifth, more preferably between one thousandth and one twentieth, or one hundredth and one fifth, or one fiftieth and one fifth, of the EC50 value of the compound at adenosine receptors at pH 7.4. More preferably the amount administered gives rise to a plasma concentration that is maintained for more than one hour at one ten thousandth to one fifth, or one ten thousandth to one twentieth, or one ten thousandth to one hundredth, most preferably one ten thousandth to one thousandth.

For the avoidance of doubt, the EC50 value of a compound is defined herein as the concentration of the compound that provokes a receptor response halfway between the baseline receptor response and the maximum receptor response (as determined, for example, using a dose-response curve).

The EC50 value should be determined under standard conditions (balanced salt solutions buffered to pH 7.4). For EC50 determinations using isolated membranes, cells and tissues this would be in buffered salt solution at pH 7.4 (e.g. cell culture medium), for example as in Daly et al. (Pharmacol. (1993) 46, 91-100), or preferably Tilburg et al (J. Med. Chem. (2002) 45, 91-100). The EC50 could also be determined *in vivo* by measuring adenosine receptor mediated responses in a normal healthy animal, or even in a tissue perfused under normal conditions (i.e. oxygenated blood, or oxygenated isotonic media, also buffered at pH 7.4) in a normal healthy animal.

Alternatively, the amount of a compound identified by a method of the invention, or of formula (I) or (II), or of Example 10, that is administered may be an amount that results in a peak plasma concentration that is one thousandth to one twentieth, one thousandth to one third, more preferably one hundredth to one fifth, or one fiftieth to one tenth, of the Kd value at adenosine receptors. More preferably the amount is an amount that results in a peak plasma concentration that is one ten thousandth to one fifth, or one ten thousandth to one twentieth, or one ten thousandth to one hundredth, most preferably one ten thousandth to one thousandth, of the Kd value at adenosine receptors.

It will be appreciated that the Kd value of the compound is likely to be different for different adenosine receptors (i.e. the A1, A2A, A2B, A3 adenosine receptors). The amount of the compound that is to be administered may be calculated relative to the lowest or highest Kd value of the compound for the different receptors.

Preferably the amount of the compound that is administered is an amount that results in a plasma concentration that is maintained for at least one hour between one thousandth and one fifth, more preferably between one thousandth and one twentieth, or one hundredth and one fifth, or one fiftieth and one fifth, of the Kd value of the compound at adenosine receptors. More preferably the amount results in a plasma concentration that is maintained for at least one hour at one ten thousandth to one fifth, or one ten thousandth to one twentieth, or one ten thousandth to one hundredth, most preferably one ten thousandth to one thousandth of the Kd value of the compound at adenosine receptors.

The Kd value of the compound at each receptor should be determined under standard conditions using plasma membranes as a source of the adenosine receptors derived either from tissues or cells endogenously expressing these receptors or from cells transfected with DNA vectors encoding the adenosine receptor genes. Alternatively whole cell preparations using cells expressing adenosine receptors can be used. Labelled ligands (e.g. radiolabelled) selective for the different receptors should be used in buffered (pH7.4) salt solutions (see e.g. Tilburg et al, J. Med. Chem. (2002) 45, 420-429) to determine the binding affinity and thus the Kd of the compound at each receptor.

Alternatively, the amount of a compound identified by a method of the invention, or of formula (I) or (II), or of Example 10, that is administered may be an amount that is one thousandth to one fifth, or one fiftieth to one third (preferably one thousandth to one twentieth, or one hundredth or one fiftieth to one fifth) of the minimum dose of the compound that gives rise to bradycardia, hypotension or tachycardia side effects in animals of the same species as the subject to which the compound is to be administered. Preferably the amount is one tenth to one fifth of the minimum dose that gives rise to the side effects. More preferably the amount is one ten thousandth to one fifth, or one ten thousandth to one twentieth, or one ten thousandth to one hundredth, most preferably one ten thousandth to one thousandth of the minimum does that gives rise to the side effects.

Preferably the amount administered gives rise to a plasma concentration that is maintained for more than 1 hour between one thousandth and one twentieth, or one hundredth or one fiftieth and one fifth of the minimum plasma concentration that gives rise to the side effects. More preferably the amount administered gives rise to a plasma concentration that is maintained for more than 1 hour at one ten thousandth to one fifth, or one ten thousandth to one twentieth, or one ten thousandth to one hundredth, most preferably one ten thousandth to one thousandth, of the minimum plasma concentration that gives rise to the side effects.

Alternatively, the amount of a compound identified by a method of the invention, or of formula (1) or (11), or of Example 10, that is administered may be an amount that gives rise to plasma concentrations that are one thousandth to one fifth, or one fiftieth to one third (preferably one thousandth to one twentieth, or one hundredth or one fiftieth to one fifth) of the minimum plasma concentration of the compound that cause bradycardia, hypotension or tachycardia side effects in animals of the same species as the subject to which the compound is to be administered. Preferably the amount gives rise to plasma concentrations that are one tenth to one fifth of the minimum plasma concentration that causes the side effects. More preferably the amount gives rise to plasma concentrations that are one ten thousandth to one fifth, or one ten thousandth to one twentieth, or one ten thousandth to one hundredth, most preferably one ten thousandth to one thousandth, of the minimum plasma concentration that causes the side effects.

Preferably the amount administered gives rise to a plasma concentration that is maintained for more than 1 hour between one thousandth and one twentieth, or one hundredth or one fiftieth and one fifth, of the minimum plasma concentration that causes the side effects. More preferably the amount gives rise to a plasma concentration that is maintained for more than one hour at one ten thousandth to one fifth, or one ten thousandth to one twentieth, or one ten thousandth to one hundredth, most preferably one ten thousandth to one thousandth, of the minimum plasma concentration that causes the side effects.

The appropriate dosage of a compound identified using a method of the invention, or of formula (I) or (II), or of Example 10, will vary with the age, sex, weight, and condition of the patient, the potency of the compound, and the route of administration, etc. The appropriate dosage can readily be determined by one skilled in the art.

It is expected that the amount of a compound identified by a method of the invention, or of formula (I) or (II), or of Example 10, that is administered should be 0.001 to 15mg/kg, or 0.01 to 15 mg/kg, for example 0.01 to 5 or 10 mg/kg, or 0.001 to 0.0 1mg/kg. The amount may be less than 6 mg/kg, preferably at least 0.001, or 0.01 or 0.05 mg/kg, for example 0.01 to 2 mg/kg. The amount may be at least 0.1 mg/kg, for example 0.1 to 1 or 2 mg/kg, or 0.2 to 1 mg/kg. A typical amount is 0.2 or 0.6 to 1.2 mg/kg.

A unit dosage of a compound identified by a method of the invention, or of formula (I) or (11), or of Example 10, typically comprises up to 500 mg (for example 1 to 500 mg, preferably 5 to 500 mg) of the active agent. Preferably the active agent is in the form of a pharmaceutical composition comprising the active agent and a physiologically acceptable carrier, excipient, or diluent. The preferred dosage is 0.1 to 2, e.g. 0.5 to 1, typically about 0.6, mg of the active agent per kg of the (human) subject. At these levels, effective treatment can be achieved substantially without a concomitant fall (for example, no more than 10%) in blood pressure.

Preferred doses for a 70kg human subject are less than 420mg, preferably at least 0.7mg, more preferably at least 3.5mg, most preferably at least 7mg. More preferably 7 to 70mg, or 14 to 70mg.

The dosage amounts specified above are significantly lower (up to approximately 1000 times lower) than would be expected (based on the EC50 value of spongosine at the adenosine A2A receptor) to be required for the compounds to have any beneficial therapeutic effect.

Compounds identified by methods of the invention, or compounds of formula (I) or (II), or of Example 10, may be administered with or without other therapeutic agents, for example anti-inflammatories (steroids, NSAIDs, methotrexate), analgesics (such as opiates, NSAIDs, cannabinoids, tachykinin modulators, or bradykinin modulators) or anti-hyperalgesics (such as gabapentin, pregabalin, cannabinoids, sodium or calcium channel modulators, anti-epileptics or anti-depressants).

In general, a compound identified by a method of the invention or a compound of formula (I) or (II), or of Example 10, may be administered by known means, in any suitable formulation, by any suitable route. A compound is preferably administered orally, parenterally, sublingually, transdermally, intrathecally, or transmucosally. Other suitable routes include intravenous, intramuscular, subcutaneous, inhaled, and topical. The amount of drug administered will typically be higher when administered orally than when administered, say, intravenously.

Suitable compositions, for example for oral administration, include solid unit dose forms, and those containing liquid, e.g. for injection, such as tablets, capsules, vials and ampoules, in which the active agent is formulated, by known means, with a physiologically acceptable excipient, diluent or carrier. Suitable diluents and carriers are known, and include, for example, lactose and talc, together with appropriate binding agents etc.

A preferred administration frequency of compounds is expected to be two or three times per day.

Compounds can also serve as a basis for identifying more effective drugs, or drugs that have further reduced side effects.

Embodiments of the invention are now described, by way of example only, with reference to the accompanying drawings in which:
Figure 1 shows the increase in affinity of 2-methoxyadenosine for the adenosine A2A receptor at pH5.5 compared to pH7.4;
Figure 2 shows that the affinity of 2-methoxyadenosine (but not CGS21680) for the rat adenosine A2A receptor increases as the pH is reduced (from 7.4 to 7.0 to 6.8);
Figure 3 shows the increase in efficacy of 2-methoxyadenosine, but not CGS21680, for the adenosine A2A receptor at pH7.0 compared to pH7.4;
Figure 4 shows that 2-methoxyadenosine inhibits the hyperalgesic effect of carageenan induced inflammation;
Figure 5 shows that 2-methoxyadenosine (0.624 mg/kg p.o.) has no significant effect on blood pressure or heart rate;
Figure 6 shows the anti-hyperalgesic action of 2-methoxyadenosine in the chronic constriction injury model of neuropathic pain;
Figure 7 shows that 2-methoxyadenosine (62.4 and 624 µg/kg i.p.) inhibits carrageenan (CGN) induced inflammation with comparable efficacy to indomethacin (3mg/kg, po), at concentrations that do not affect blood pressure; and
Figure 8 shows the change in plasma concentration over time after administration of 2-methoxyadenosine (0.6 mg/kg) to a rat.

### Example 1

### The affinity of 2-methoxyadenosine for the adenosine A2A receptor increases as pH is reduced

Rat striatal membranes were incubated for 90 minutes at 22°C in the presence of 2nM [3H]-CGS21680, 1Unit/ml adenosine deaminase and increasing concentrations of 2-methoxyadenosine, prior to filtration and liquid scintillation counting. The data were fitted to one and two site binding curves (see Figure 1):
a) represents the total amount of specific binding as a percentage;
b) represents the proportion of the sites in the high affinity state;
c) represents the Ki of the high affinity state (N.B. at pH 7.4, the Hill slope (nH) was close to unity and the curve fitting algorithm for a two site fit was unable to define realistic properties to a high affinity state); and
d) represents the Ki of the low affinity state.

### Example 2

### Comparison of the affinity of 2-methoxyadenosine and CGS21680 for the adenosine A2A receptor at different pH

Displacement of [3H]-CGS21680 binding was performed as described for Example 1. The results, shown in Figure 2, demonstrate that the affinity of 2-methoxyadenosine (but not CGS21680) for the rat adenosine A2A receptor increases as the pH is reduced (from 7.4 to 7.0 to 6.8).

### Example 3

### The efficacy of 2-methoxyadenosine, but not CGS21680, for the adenosine A2A receptor is increased at pH7.0 compared to pH7.4

The human A2A receptor was expressed in HEK293 cells, and the ability of agonists to stimulate cAMP accumulation assessed in the presence of rolipram to inhibit phosphodiesterase enzymes. The results are shown in Figure 3.

### Example 4

### 2-methoxyadenosine inhibits the hyperalgesic effect of carageenan induced inflammation

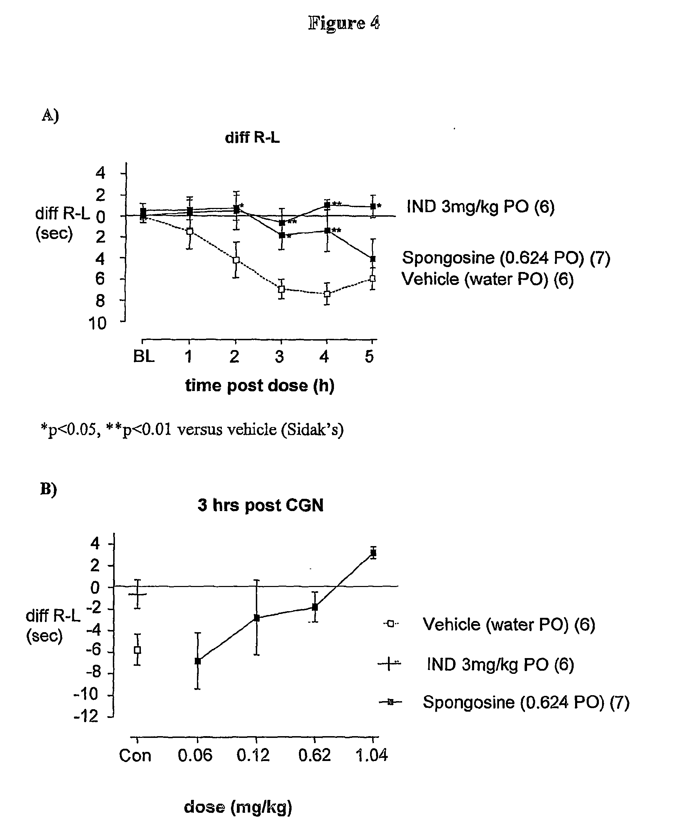

Figure 4 shows that 2-methoxyadenosine inhibits the hyperalgesic effect of carageenan induced inflammation: A. 2-methoxyadenosine (0.6 mg/kg) inhibits carrageenan (CGN) induced thermal hyperalgesia (CITH) with comparable efficacy to indomethacin (3mg/kg, po). B. Concentration-response relationship for 2-methoxyadenosine at 3 hrs post dosing.

Carrageenan (2%, 10 microlitres) was administered into the right hind paw. A heat source was placed close to the treated and untreated hind paws, and the difference in the paw withdrawal latencies is shown. 2-methoxyadenosine was administered at the same time as carrageenan. 2-methoxyadenosine was as effective as indomethacin (Indo, 3mg/kg p.o.).

### Example 5

### 2-methoxyadenosine (at 0.624 mg/kg p.o.) has no significant effect on blood pressure or heart rate

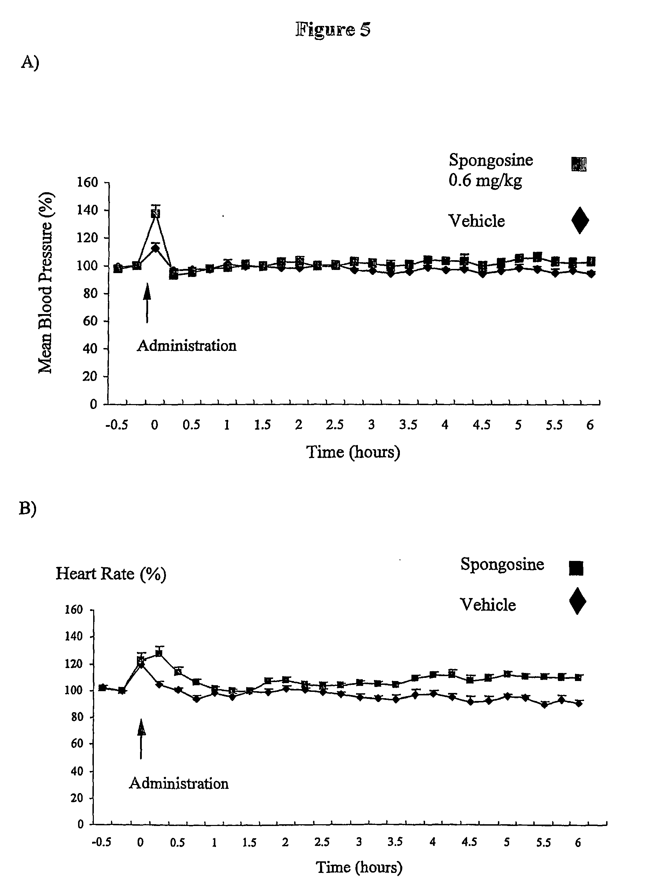

An implantable radiotelemetry device was placed in the abdominal cavity of 6 rats per group. The pressure catheter of the device was inserted in the abdominal aorta and two electrodes tunnelised under the skin in a lead II position (left side of abdominal cavity/right shoulder). Individual rats were placed in their own cage on a radioreceptor (DSI) for data acquisition. The effect of 0.6mg/kg 2 methoxyadenosine or vehicle (p.o.) on blood pressure was then assessed. The results are shown in Figure 5: A: blood pressure; B: heart rate.

### Example 6

### The anti-hyperalgesic action of 2-methoxyadenosine in the chronic constriction injury model of neuropathic pain

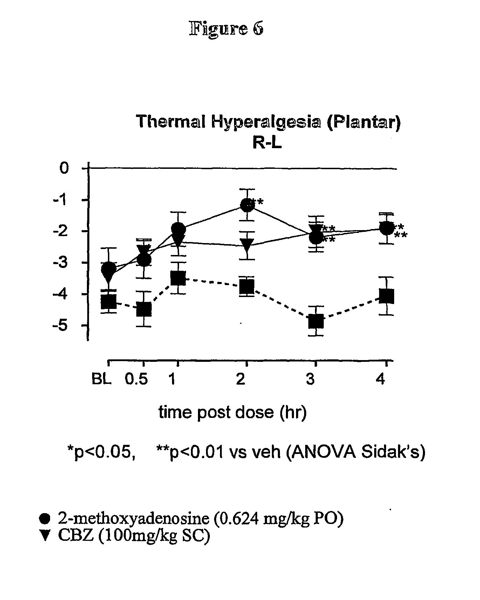

2-methoxyadenosine (0.624mg/kg p.o.) inhibits thermal hyperalgesia caused by chronic constriction injury of the rat sciatic nerve. Under anaesthesia the sciatic nerve was displayed in the right leg, and four loose ligatures tied round the nerve bundle. After approximately two weeks the rats developed thermal hyperalgesia in the operated leg as judged by the difference in paw withdrawal latencies of the right and left paws. Administration of 2-methoxyadenosine reduced the hyperalgesia as shown by the reduction in the difference between the withdrawal latencies. 2-methoxyadenosine was as, or more, effective than carbamazepine (CBZ, 100mg/kg s.c.). The results are shown in Figure 6.

### Example 7

### 2-methoxyadenosine (62.4 and 624 µg/kg i.p.) inhibits carrageenan (CGN) induced inflammation with comparable efficacy to indomethacin (3mg/kg. po), at concentrations that do not affect blood pressure

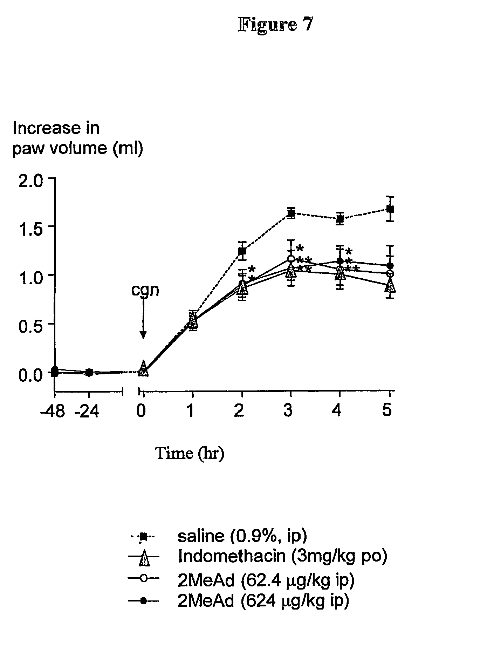

Carrageenan (2%, 10 microlitres) was administered into the right hind paw, and the paw volume assessed by plethysomometry. 2-methoxyadenosine was administered at the same time as carrageenan. The results are shown in Figure 7. 2-methoxyadenosine was as effective as indomethacin (Indo, 3mg/kg p.o.).

### Example 8

### The change in plasma concentration over time after administration of 2-methoxyadenosine (0.6 mg/kg) to a rat

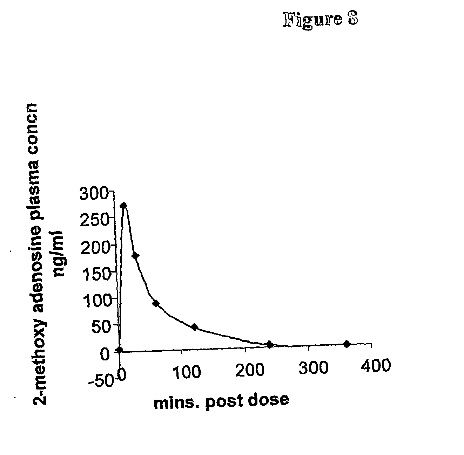

The EC50 value of 2-methoxyadenosine at adenosine receptors (measured at pH7.4) is 900ng/ml (3 µM). It can be seen from Figure 8 that the plasma concentration remains above 2% of the EC50 value for more than 3 hours. Anti-inflammatory and anti-hyperalgesic effects have been observed (without blood pressure changes) when the peak plasma concentration is between 1% and 30°l° of the EC50 value determined *in vitro.* If the peak plasma concentration reaches the EC50 value profound reductions in blood pressure occur that last for hours.

### Example 9

The increased affinity of adenosine receptor agonists at lower pH was associated with a corresponding increase in GTP shift, suggesting that these agonists were also more efficacious at the lower pH. These results are summarised below.

**Table 1. Displacement of 3H-CGS21680 from the rat striatal A2A receptor. (values are Ki in nM)**

| | pH 7.4 | pH5.5 |
|---|---|---|
| CGS21680 | 10.5 ± 2.1 | 2.8 ± 1.0 |
| NECA | 3.5 ± 0.9 | 1.1 ± 0.2 |
| CV1808 | 170 ± 20 | 35 ± 9 |
| R-PIA | 172 ± 21 | 21 ± 1.4 |
| S-PIA | 1712 ± 423 | 271 ± 4.9 |

| | | |
|---|---|---|
| NECA: 5' N-ethylcarboxamidoadensosine; CV1808: 2-phenylamino-adenosine; PIA: phenylisopropyladenosine. | | |

All of the above revealed only one affinity state for agonists, suggesting that the high and low affinity states are similar in affinity. However 2-methoxyadenosine and 2-ethoxyadenosine revealed two apparent affinity states at the lower pH (Table 2). The high affinity states corresponded to approximately 25% of the total number of binding sites, and were abolished by the presence of GTP, suggesting that they correspond to the high affinity agonist binding state.

**Table 2. Displacement of 3H-CGS21680 from the rat striatal A2A receptor. (values are Ki in nM)**

| 2-methoxyadenosine | |
|---|---|
| pH 7.4: High affinity not detectable | Low affinity 2500 ± 200 |
| pH 5.5: High affinity 12.1 ± 2.7 | Low affinity 1200 ± 100 |

| 2-ethoxyadenosine | |
|---|---|
| pH 7.4: High affinity 245 ± 76 | Low affinity 6600 ± 2200 |
| pH 5.5: High affinity 2.8 ± 1.2, | Low affinity 1200 ± 120 |

In addition compounds were discovered which showed higher affinity for the rat A1 receptor at lower pH (Table 3).

**Table 3. Displacement of 3H-DPCPX from the rat cortex A1 receptor (Ki nM).**

| 2-chloroadenosine | |
|---|---|
| pH 7.4 High affinity 4.4 ± 1.8 | Low affinity 1670 ± 346 |
| pH 6.2 High affinity 3.7 ± 0.5 | Low affinity 1660 ± 40 |

| 3'-deoxy-2-chloroadenosine | |
|---|---|
| pH 7.4: High affinity 227 ± 74 | Low affinity 11,500 ± 850 |
| pH 6.2: High affinity 17 ± 46 | Low affinity 13,400 ± 2400 |

At both pH values the stable analogue of GTP (GppNHp) abolished the high affinity state recognised by 3'-deoxy-2-chloroadenosine.

### Example 10

### Compounds found to have higher affinity for adenosine A2A receptors at lower pH

The compounds listed below were found to have higher affinity for adenosine A2A receptors at pH 5.5 than pH7.4. The A2A receptor binding assay was carried out using [3H]-CGS21680 and analysed as described for Example 1. The Ki values of the high and low affinity binding sites detected at pH5.5 are indicated, and the Ki of the site detected at pH7.4. 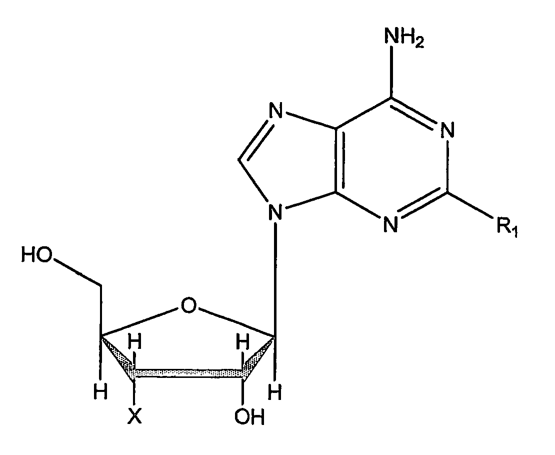
**When X = OH**

| **Structure R₁** | **pH 5.5 (Ki 1) nM** | **pH 5.5 (Ki 2) nM** | **pH 7.4 (Ki 2) nM** |
|---|---|---|---|
| OCH3 | 1.5 | 380 | 1300 |
| OCH₂CH₂CH₂CH₃ | 11 | 560 | 280 |
| O CH₂CH₂CH₂CH₂CH₂CH₃ | 3 | 170 | 1500 |
| OPh | 71 | 1200 | 2500 |
| O-(4-cyano)Ph | 4 | 380 | 1300 |
| O-(3-Ph)Ph | 0.7 | 135 | 620 |
| 5-indanyloxy | 12 | 175 | 760 |
| O-(3-CH(CH₃)₂)Ph | 16 | 240 | 560 |
| NH(CH₃) | 24 | 2240 | 1356 |
| NHCH₂CH₃ | 130 | 3500 | 1200 |
| N(CH₃)₂ | 24 | 21440 | 13350 |
| NHCH₂CH₂CH₂CH₂CH₂CH₃ | 0.7 | 20 | 290 |
| NHPh | 5 | 2028 | 160 |
| NH-(4-MeO)Ph | 3 | 180 | 55 |
| NH-(4-F)Ph | 10 | 150 | 200 |
| NH-cyclopentyl | 2 | 60 | 420 |
| NH-cyclohexyl | 0.4 | 100 | 1000 |
| N-CH₃, N-CH₂CH₂CH(CH₃)₂ | 26 | 2600 | 4000 |
| OCH₂cyclopentyl | 0.2 | 54 | 200 |
| S0₂CH₂CH₃ | 100 | 5250 | 39000 |
| OCH₂CH₂OH | 4 | 164 | 203 |
| CH₂CH₂CH₂CH₂CH₂CH₂CH₃ | 15 | 630 | 800 |

**X=H**

| **Structure R₁** | **pH 5.5 (Ki 1) nM** | **pH 5.5 (ki 2) nM** | **pH 7.4 (Ki 2) nM** |
|---|---|---|---|
| O CH₂CH₂CH₂CH₂CH₂CH₃ | 13 | 440 | 2990 |

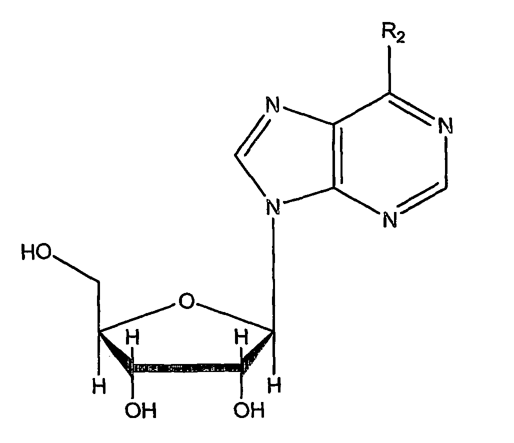

| **Structure R²** | **pH 5.5 (Ki1) nM** | **pH 5.5 (Ki2) nM** | **pH 7.4 (Ki 2) nM** |
|---|---|---|---|
| N(CH₃)₂ | 42 | 9400 | 450000 |
| NHCH₂CHC(CH₃)₂ | 1.5 | 380 | 8600 |
| N-CH₃, N-CH₂Ph | 7 | 2100 | 18500 |
| Piperazinyl | 38 | 7000 | 5000 |
| N-Me, N-(CH₂CH₂OCH₃) | 13 | 6470 | 13000 |

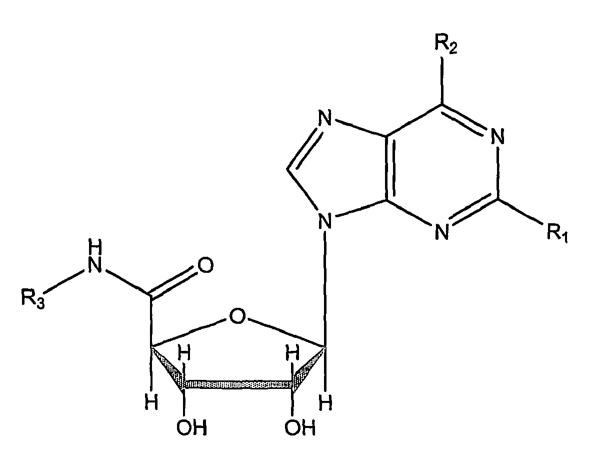

| **R₁** | **R₂** | **R₃** | **pH 5.5 (Ki 1) nM** | **pH 5.5 (Ki 2) nM** | **pH 7.4 (Ki 2) nM** |
|---|---|---|---|---|---|
| H | NH₂ | CH(CH₃)₂ | 5 | 190 | 1930 |
| H | NH₂ | H | 9 | 180 | 270 |
| H | NHCH₃ | CH(CH₃)₂ | 188 | 3420 | 2440 |
| OCH₃ | NH₂ | Ph | 230 | 53400 | 26100 |

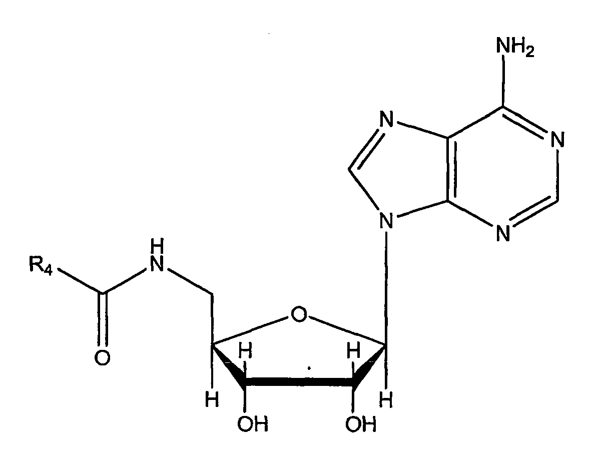

| **Structure R₄** | **pH 5.5 (Ki 1) nM** | **pH 5.5 (Ki 2) nM** | **pH 7.4 (Ki 2) nM** |
|---|---|---|---|
| CH₂CH₂CH₃ | 145 | 53550 | 16900 |
| NHCH₂CH₃ | 40 | 5900 | 6570 |

## Claims

1. A method for identifying a potential therapeutic agent, which comprises determining the affinity of a test compound for an adenosine receptor at a higher pH of at least 7.4, and also at a lower pH from 5.5 to 7.2, or from 5.0 to 7.0, and identifying the compound as a said agent if the affinity at the lower pH is over 10 times greater than the affinity at the higher pH.

2. A method according to claim 1 wherein determining the affinity of the test compound comprises measuring binding of different concentrations of labelled test compound to the receptor.

3. A method according to claim 1 wherein determining the affinity of the test compound comprises measuring displacement of a bound labelled compound from the adenosine receptor with unlabelled test compound.

4. A method according to any preceding claim wherein the adenosine receptor is in a tissue, a tissue slice, an intact cell, a disrupted cell, or a cell derived membrane.

5. A method for identifying a potential therapeutic agent, which comprises determining the efficacy of action of a test compound at an adenosine receptor at a higher pH of at least 7.4, and also at a lower pH from 5.5 to 7.2, or from 5.0 to 7.0, and identifying the compound as a said agent if the efficacy at the lower pH is over 10 times greater than the efficacy at the higher pH.

6. A method according to claim 5, wherein determining the efficacy of the test compound comprises contacting the test compound with a tissue, tissue slice, intact cell or partially disrupted cell comprising the adenosine receptor, and determining adenosine receptor action by measuring accumulation or depletion of a signalling molecule in the tissue, tissue slice, intact cell or partially disrupted cell.

7. A method according to claim 6 wherein the signalling molecule is cAMP, IP3 or free calcium.

8. A method according to claim 5, wherein determining the efficacy of the test compound comprises contacting the test compound with a biological membrane comprising the adenosine receptor, and determining adenosine receptor action by measuring: activation of a G protein; activity of an enzyme; or ion flow through an ion channel associated with the biological membrane.

9. A method according to claim 8, wherein activation of the G protein is measured by use of a radiolabelled guanine nuceotide, or wherein the enzyme activity measured is adenylyl cyclase, phosphodiesterase, phospholipase or protein kinase activity, or wherein the ion channel through which ion flow is measured is a calcium or a potassium channel.

10. A method according to claim 5, wherein determining the efficacy of the test compound comprises contacting the test compound with a tissue, tissue slice, intact cell, disrupted cell or cell derived membrane comprising the adenosine receptor, and determining adenosine receptor action by measuring protein kinase activity in the tissue, tissue slice, intact cell, disrupted cell or cell derived membrane.

11. A method according to claim 5, wherein determining the efficacy of the test compound comprises contacting the test compound with a tissue, tissue slice, intact cell, disrupted cell or cell derived membrane comprising the adenosine receptor, and determining adenosine receptor action by measuring phospholipase activity (e.g. phospholipase C, phospholipase A2, phospholipase D) in the tissue, tissue slice, intact cell, disrupted cell or cell derived membrane.

12. A method according to claim 5, wherein determining the efficacy of the test compound comprises contacting the test compound with a tissue, tissue slice, intact cell, disrupted cell or cell derived membrane comprising the adenosine receptor, and determining adenosine receptor action by measuring protein phosphorylation and/or dephosphorylation in the tissue, tissue slice, intact cell, disrupted cell or cell derived membrane.

13. A method according to any preceding claim, wherein the lower pH is from 5.5 to 6.5.

14. A method according to any preceding claim, wherein the lower pH is from 5.5 to 6.2.

15. A method according to any preceding claim, wherein the adenosine receptor is an adenosine A2A receptor, an adenosine A1 receptor, or an adenosine A3 receptor.

16. A method according to any preceding claim which further comprises determining whether the identified agent has a therapeutic effect.

17. A method according to claim 16 in which a non human animal model is used to determine whether the identified agent has a therapeutic effect.

18. A method according to claim 16 or 17, wherein it is determined whether the identified agent has a therapeutic effect against pain or inflammation.

19. A method according to any of claims 16 to 18, wherein it is determined whether the identified agent has a therapeutic effect against cancer, ischemia-reperfusion injury, excessive neuronal activity (for example in epilepsy, or chronic pain or hyperlagesia, including inflammatory and neuropathic pain), sepsis, septic shock, neurodegeneration (including Alzheimer's Disease), muscle fatigue or muscle cramp.

20. A method according to any of claims 16 to 19, wherein it is determined whether the identified agent has a therapeutic effect against arthritis, asthma, psoriasis, bowel inflammation, rheumatoid arthritis, irritable bowel syndrome or osteoarthritis.

21. A method according to any of claims 16 to 19, wherein it is determined whether the identified agent has a therapeutic effect against hyperalgesia caused as a result of neuropathy, including bowel pain, back pain, cancer pain, HIV pain, phantom limb pain, post-operative pain, diabetic neuropathy, polyneuropathy, post-herpes neuralgia, or trigeminal neuralgia.

22. A method according to any of claims 16 to 19, wherein it is determined whether the identified agent has a therapeutic effect against hyperalgesia caused as a result of inflammatory disease, including bowel pain, back pain, cancer pain, fibromyalgia, post-operative pain, osteoarthritis, or rheumatoid arthritis.

23. A method according to any of claims 16 to 22 in which the therapeutic effect of the identified agent is determined at a concentration below the EC50 value, preferably one ten thousandth to one fifth of the EC50 value, of the agent at the adenosine receptor at pH 7.4.

24. A method according to any of claims 1 E to 23 in which the side effects caused by the identified agent are determined.

25. A method according to claim 24 in which the side effects are determined at a concentration below the EC50 value, preferably one ten thousandth to one fifth of the EC50 value, of the agent at the adenosine receptor at pH 7.4.

26. A method according to claim 24 or 25 in which the side effects are bradycardia, hypotension or tachycardia side effects.

27. A method for identifying a potential therapeutic agent which comprises contacting a test compound with an adenosine receptor in a pathological tissue, cell, or membrane and a corresponding normal tissue, cell, or membrane at a concentration below the EC50 value of the test compound at the adenosine receptor at pH 7.4, and determining whether there is any difference in action of the adenosine receptor in response to contact with the test compound between the normal tissue and the pathological tissue.

28. A method according to claim 27, wherein the test compound is identified as a potential therapeutic agent if the test compound is an agonist of the adenosine receptor, and the action of the adenosine receptor in response to the test compound is greater in the pathological tissue than the normal tissue.

29. A method according to claim 27 or 28, wherein the pathological tissue comprises epithelial tissue.

30. A method according to claim 29 for identifying a potential therapeutic agent for treating psoriasis, asthma, or COPD.

## Patentansprüche

1. Verfahren zum Identifizieren eines potenziellen Therapeutikums, umfassend das Bestimmen der Affinität einer Testverbindung für einen Adenosinrezeptor bei einem höheren pH-Wert von mindestens 7,4 und auch bei einem niedrigeren pH-Wert von 5,6 bis 7,2 oder von 5,0 bis 7,0 und Identifizieren der Verbindung als ein oben genanntes Mittel, wenn die Affinität bei dem niedrigeren pH-Wert mehr als das 10-Fache beträgt als die Affinität bei dem höheren pH-Wert.

2. Verfahren gemäß Anspruch 1, wobei das Bestimmen der Affinität der Testverbindung das Messen des Bindens von verschiedenen Konzentrationen einer markierten Testverbindung an den Rezeptor umfasst.

3. Verfahren gemäß Anspruch 1, wobei das Bestimmen der Affinität der Testverbindung das Messen der Verdrängung einer gebundenen markierten Verbindung von dem Adenosinrezeptor mit unmarkierter Testverbindung umfasst.

4. Verfahren gemäß mindestens einem der vorhergehenden Ansprüche, wobei der Adenosinrezeptor sich in einem Gewebe, einem Gewebeschnitt, einer intakten Zelle, einer aufgebrochenen Zelle oder einer von einer Zelle herrührenden Membran befindet.

5. Verfahren zum Identifizieren eines potenziellen Therapeutikums, umfassend das Bestimmen der Effizienz der Wirkung einer Testverbindung bei einem Adenosinrezeptor bei einem höheren pH-Wert von mindestens 7,4 und auch bei einem niedrigeren pH-Wert von 5,5 bis 7,2 oder von 5,0 bis 7,0, und Identifizieren der Verbindung als ein oben genanntes Mittel, wenn die Wirksamkeit bei dem niedrigeren pH-Wert mehr als das 10-Fache beträgt als die Wirksamkeit bei dem höheren pH-Wert.

6. Verfahren gemäß Anspruch 5, wobei das Bestimmen der Wirksamkeit der Testverbindung das Kontaktieren der Testverbindung mit einem Gewebe, einem Gewebeschnitt, einer intakten Zelle oder einer den Adenosinrezeptor umfassenden teilweise aufgebrochenen Zelle umfasst, und Bestimmen der Adenosinrezeptor-Wirkung durch Messen der Akkumulation oder Verarmung eines signalgebenden Moleküls in dem Gewebe, dem Gewebeschnitt, der intakten Zelle oder der teilweise aufgebrochenen Zelle.

7. Verfahren gemäß Anspruch 6, wobei das signalgebende Molekül cAMP, IP3 oder freies Calcium ist.

8. Verfahren gemäß Anspruch 5, wobei das Bestimmen der Wirksamkeit der Testverbindung das Kontaktieren der Testverbindung mit einer den Adenosinrezeptor umfassenden biologischen Membran und das Bestimmen der Adenosinrezeptor-Wirkung durch Messen: der Aktivierung eines G-Proteins; der Aktivität eines Enzyms; oder des Ionenflusses durch einen mit der biologischen Membran assoziierten Ionenkanal umfasst.

9. Verfahren gemäß Anspruch 8, wobei die Aktivierung des G-Proteins durch die Verwendung eines radiomarkierten Guaninnukleotids gemessen wird, oder wobei die gemessene Enzymaktivität Adenylylcyclase-, Phosphodiesterase-, Phospholipase- oder Proteinkinaseaktivität ist, oder wobei der Ionenkanal, durch welchen der Ionenstrom gemessen wird, ein Calcium- oder Kaliumkanal ist.

10. Verfahren gemäß Anspruch 5, wobei das Bestimmen der Wirksamkeit der Testverbindung das Kontaktieren der Testverbindung mit einem Gewebe, einem Gewebeschnitt, einer intakten Zelle, einer aufgebrochenen Zelle oder einer den Adenosinrezeptor umfassenden, von Zellen abgeleiteten Membran und das Bestimmen der Adenosinrezeptor-Wirkung durch Messen der Proteinkinaseaktivität in dem Gewebe, dem Gewebeschnitt, der intakten Zelle, der aufgebrochenen Zelle oder der von Zellen abgeleiteten Membran umfasst.

11. Verfahren gemäß Anspruch 5, wobei das Bestimmen der Wirksamkeit der Testverbindung das Kontaktieren der Testverbindung mit einem Gewebe, einem Gewebeschnitt, einer intakten Zelle, einer aufgebrochenen Zelle oder einer den Adenosinrezeptor umfassenden, von Zellen abgeleiteten Membran und das Bestimmen der Adenosinrezeptor-Wirkung durch Messen der Phospholipaseaktivität (z. B. Phospholipase C, Phospholipase A2, Phospholipase D) in dem Gewebe, dem Gewebeschnitt, der intakten Zelle, der aufgebrochenen Zelle oder der von Zellen abgeleiteten Membran umfasst.

12. Verfahren gemäß Anspruch 5, wobei das Bestimmen der Wirksamkeit der Testverbindung das Kontaktieren der Testverbindung mit einem Gewebe, einem Gewebeschnitt, einer intakten Zelle, einer aufgebrochenen Zelle oder einer den Adenosinrezeptor umfassenden, von Zellen abgeleiteten Membran und das Bestimmen der Adenosinrezeptor-Wirkung durch Messen der Proteinphosphorylierung und/oder Dephosphorylierung in dem Gewebe, dem Gewebeschnitt, der intakten Zelle, der aufgebrochenen Zelle oder der von Zellen abgeleiteten Membran umfasst.

13. Verfahren gemäß mindestens einem der vorhergehenden Ansprüche, wobei der niedrigere pH-Wert 5,5 bis 6,5 beträgt.

14. Verfahren gemäß mindestens einem der vorhergehenden Ansprüche, wobei der niedrigere pH-Wert 5,5 bis 6,2 beträgt.

15. Verfahren gemäß mindestens einem der vorhergehenden Ansprüche, wobei der Adenosinrezeptor ein Adenosin-A2A-Rezeptor, ein Adenosin-A1-Rezeptor oder ein Adenosin-A3-Rezeptor ist.

16. Verfahren gemäß mindestens einem der vorhergehenden Ansprüche, weiterhin umfassend die Bestimmung, ob das identifizierte Agens eine therapeutische Wirkung besitzt.

17. Verfahren gemäß Anspruch 16, in welchem ein Nicht-Humanes-Tiermodell für die Bestimmung verwendet wird, ob das identifizierte Agens eine therapeutische Wirkung besitzt.

18. Verfahren gemäß Anspruch 16 oder 17, wobei bestimmt wird, ob das identifizierte Agens eine therapeutische Wirkung gegen Schmerz oder Entzündung besitzt.

19. Verfahren gemäß mindestens einem der vorhergehenden Ansprüche 16 bis 18, wobei bestimmt wird, ob das identifizierte Agens eine therapeutische Wirkung gegen Krebs, Ischämie-Reperfusionsschädigung, übermäßige neuronale Aktivität (zum Beispiel bei Epilepsie oder chronischem Schmerz oder Hyperalgesie, einschließlich entzündlichem und neuropathischem Schmerz), Sepsis, septischen Schock, Neurodegeneration (einschließlich Alzheimer-Krankheit), Muskelermüdung oder Muskelkrampf besitzt.

20. Verfahren gemäß mindestens einem der vorhergehenden Ansprüche 16 bis 19, wobei bestimmt wird, ob das identifizierte Agens eine therapeutische Wirkung gegen Arthritis, Asthma, Psoriasis, Darmentzündung, rheumatoide Arthritis, Reizdarmsyndrom oder Osteoarthritis besitzt.

21. Verfahren gemäß mindestens einem der vorhergehenden Ansprüche 16 bis 19, wobei bestimmt wird, ob das identifizierte Agens eine therapeutische Wirkung gegen Hyperalgesie besitzt, die als eine Folge von Neuropathie, einschließlich Darmschmerz, Rückenschmerz, Krebsschmerz, HIV-Schmerz, Phantom-Gliederschmerz, postoperativen Schmerz, diabetische Neuropathie, Polyneuropathie, Post-Herpes-Neuralgie oder trigeminale Neuralgie, verursacht wird.

22. Verfahren gemäß mindestens einem der vorhergehenden Ansprüche 16 bis 19, wobei bestimmt wird, ob das identifizierte Agens eine therapeutische Wirkung gegen Hyperalgesie besitzt, die als eine Folge einer entzündlichen Erkrankung, einschließlich Darmschmerz, Rückenschmerz, Krebsschmerz, Fibromyalgie, postoperativer Schmerz, Osteoarthritis oder rheumatoide Arthritis, verursacht wird.

23. Verfahren gemäß mindestens einem der Ansprüche 16 bis 22, in welchem die therapeutische Wirkung des identifizierten Agens bei einer Konzentration unter dem EC50-Wert, vorzugsweise ein Zehntausendstel bis ein Fünftel des EC50-Werts des Agens bei dem Adenosinrezeptor bei einem pH-Wert 7,4 bestimmt wird.

24. Verfahren gemäß mindestens einem der Ansprüche 16 bis 23, in welchem die durch das identifizierte Agens verursachten Nebenwirkungen bestimmt werden.

25. Verfahren gemäß Anspruch 24, in welchem die Nebenwirkungen bei einer Konzentration unter dem EC50-Wert, vorzugsweise ein Tausendstel bis ein Fünftel des EC50-Werts, des Agens bei dem Adenosinrezeptor bei einem pH-Wert von 7,4 bestimmt wird.

26. Verfahren gemäß Anspruch 24 oder 25, in welchem die Nebenwirkungen Bradykardie-, Hypotonie- oder Tachykardie-Nebenwirkungen sind.

27. Verfahren zum Identifizieren eines potenziellen Therapeutikums, umfassend das Kontaktieren einer Testverbindung mit einem Adenosinrezeptor in einem pathologischen Gewebe, einer Zelle oder einer Membran und einem/einer entsprechenden normalen Gewebe, Zelle oder Membran bei einer Konzentration unter dem EC50-Wert der Testverbindung bei dem Adenosinrezeptor bei einem pH-Wert von 7,4, und Bestimmen, ob es einen Unterschied in der Wirkung des Adenosionrezeptors als Reaktion auf einen Kontakt mit der Testverbindung zwischen dem normalen Gewebe und dem pathologischen Gewebe gibt.

28. Verfahren gemäß Anspruch 27, wobei die Testverbindung als ein potenzielles Therapeutikum identifiziert wird, wenn die Testverbindung ein Agonist des Adenosinrezeptors ist und die Wirkung des Adenosinrezeptors in Reaktion auf die Testverbindung bei dem pathologischen Gewebe größer als bei dem normalen Gewebe ist.

29. Verfahren gemäß Anspruch 27 oder 28, wobei das pathologische Gewebe Epithelgewebe umfasst.

30. Verfahren gemäß Anspruch 29 zum Identifizieren eines potenziellen Therapeutikums für die Behandlung von Psoriasis, Asthma oder COPD.

## Revendications

1. Procédé pour identifier un agent thérapeutique potentiel qui comprend la détermination de l'affinité d'un composé test pour un récepteur d'adénosine à un pH supérieur d'au moins 7,4, et aussi à un pH inférieur de 5,5 à 7,2, ou de 5,0 à 7,0, et l'identification du composé comme ledit agent si l'affinité au pH inférieur est plus de 10 fois plus grande que l'affinité au pH supérieur.

2. Procédé selon la revendication 1 où la détermination de l'affinité du composé test comprend la mesure de la liaison de différentes concentrations de composé test marqué au récepteur.

3. Procédé selon la revendication 1 où la détermination de l'affinité du composé test comprend la mesure du déplacement d'un composé marqué lié du récepteur d'adénosine avec un composé test non marqué.

4. Procédé selon l'une quelconque des revendications précédentes où le récepteur d'adénosine est dans un tissu, une coupe de tissu, une cellule intacte, une cellule dissociée ou une membrane dérivée d'une cellule.

5. Procédé pour identifier un agent thérapeutique potentiel qui comprend la détermination de l'efficacité d'action d'un composé test à un récepteur d'adénosine à un pH supérieur d'au moins 7,4, et aussi à un pH inférieur de 5,5 à 7,2, ou de 5,0 à 7,0, et l'identification du composé comme ledit agent si l'efficacité au pH inférieur est plus de 10 fois plus grande que l'efficacité au pH supérieur.

6. Procédé selon la revendication 5 où la détermination de l'efficacité du composé test comprend la mise en contact du composé test avec un tissu, une coupe de tissu, une cellule intacte ou une cellule partiellement dissociée comprenant le récepteur d'adénosine, et la détermination de l'action du récepteur d'adénosine par mesure de l'accumulation ou de l'appauvrissement d'une molécule de signalisation dans le tissu, la coupe de tissu, la cellule intacte ou la cellule partiellement dissociée.

7. Procédé selon la revendication 6 où la molécule de signalisation est cAMP, IP3 ou le calcium libre.

8. Procédé selon la revendication 5 où la détermination de l'efficacité du composé test comprend la mise en contact du composé test avec une membrane biologique comprenant le récepteur d'adénosine, et la détermination de l'action du récepteur d'adénosine par mesure de : l'activation d'une protéine G ; l'activité d'une enzyme ; ou le flux d'ions dans un canal ionique associé avec la membrane biologique.

9. Procédé selon la revendication 8 où l'activation de la protéine G est mesurée au moyen d'un nucléotide à guanine radiomarqué, ou bien où l'activité d'une enzyme mesurée est une activité adénylyl cyclase, phosphodiestérase, phospholipase ou protéine kinase, ou bien où le canal ionique dans lequel le flux d'ions est mesuré est un canal calcium ou potassium.

10. Procédé selon la revendication 5 où la détermination de l'efficacité du composé test comprend la mise en contact du composé test avec un tissu, une coupe de tissu, une cellule intacte, une cellule dissociée ou une membrane dérivée d'une cellule comprenant le récepteur d'adénosine, et la détermination de l'action du récepteur d'adénosine par mesure d'une activité protéine kinase dans le tissu, la coupe de tissu, la cellule intacte, la cellule dissociée ou la membrane dérivée d'une cellule.

11. Procédé selon la revendication 5 où la détermination de l'efficacité du composé test comprend la mise en contact du composé test avec un tissu, une coupe de tissu, une cellule intacte, une cellule dissociée ou une membrane dérivée d'une cellule comprenant le récepteur d'adénosine, et la détermination de l'action du récepteur d'adénosine par mesure d'une activité phospholipase (par exemple phospholipase C, phospholipase A2, phospholipase D) dans le tissu, la coupe de tissu, la cellule intacte, la cellule dissociée ou la membrane dérivée d'une cellule.

12. Procédé selon la revendication 5 où la détermination de l'efficacité du composé test comprend la mise en contact du composé test avec un tissu, une coupe de tissu, une cellule intacte, une cellule dissociée ou une membrane dérivée d'une cellule comprenant le récepteur d'adénosine, et la détermination de l'action du récepteur d'adénosine par mesure de la phosphorylation et/ou déphosphorylation de protéines dans le tissu, la coupe de tissu, la cellule intacte, la cellule dissociée ou la membrane dérivée d'une cellule.

13. Procédé selon l'une quelconque des revendications précédentes où le pH inférieur est de 5,5 à 6,5.

14. Procédé selon l'une quelconque des revendications précédentes où le pH inférieur est de 5,5 à 6,2.

15. Procédé selon l'une quelconque des revendications précédentes où le récepteur d'adénosine est un récepteur d'adénosine A2A, un récepteur d'adénosine A1 ou un récepteur d'adénosine A3.

16. Procédé selon l'une quelconque des revendications précédentes qui comprend en outre la détermination de ce que l'agent identifié a un effet thérapeutique.

17. Procédé selon la revendication 16 où un modèle animal non humain est utilisé pour déterminer si l'agent identifié a un effet thérapeutique.

18. Procédé selon la revendication 16 ou 17 où on détermine si l'agent identifié a un effet thérapeutique contre la douleur ou l'inflammation.

19. Procédé selon l'une quelconque des revendications 16 à 18 où on détermine si l'agent identifié a un effet thérapeutique contre le cancer, les lésions d'ischémie-reperfusion, l'activité neuronale excessive (par exemple dans l'épilepsie, ou la douleur chronique ou l'hyperalgésie, incluant la douleur inflammatoire et neuropathique), la septicémie, le choc septique, la neurodégénérescence (incluant la maladie d'Alzheimer), la fatigue musculaire ou les crampes musculaires.

20. Procédé selon l'une quelconque des revendications 16 à 19 où on détermine si l'agent identifié a un effet thérapeutique contre l'arthrite, l'asthme, le psoriasis, l'inflammation intestinale, la polyarthrite rhumatoïde, le syndrome du côlon irritable ou l'arthrose.

21. Procédé selon l'une quelconque des revendications 16 à 19 où on détermine si l'agent identifié a un effet thérapeutique contre l'hyperalgésie causée par suite d'une neuropathie, incluant la douleur intestinale, la douleur dorsale, la douleur due au cancer, la douleur due à VIH, l'algo-hallucinose, la douleur post-opératoire, la neuropathie diabétique, la polyneuropathie, la névralgie post-herpétique ou la névralgie essentielle du trijumeau.

22. Procédé selon l'une quelconque des revendications 16 à 19 où on détermine si l'agent identifié a un effet thérapeutique contre l'hyperalgésie causée par suite d'une maladie inflammatoire, incluant la douleur intestinale, la douleur dorsale, la douleur due au cancer, la fibromyalgie, la douleur post-opératoire, l'arthrose ou la polyarthrite rhumatoïde.

23. Procédé selon l'une quelconque des revendications 16 à 22 où l'effet thérapeutique de l'agent identifié est déterminé à une concentration inférieure à la valeur EC50, de préférence un dix millième à un cinquième de la valeur EC50, de l'agent au récepteur d'adénosine à pH 7,4.

24. Procédé selon l'une quelconque des revendications 16 à 23 où les effets secondaires causés par l'agent identifié sont déterminés.

25. Procédé selon la revendication 24 où les effets secondaires sont déterminés à une concentration inférieure à la valeur EC50, de préférence un dix millième à un cinquième de la valeur EC50, de l'agent au récepteur d'adénosine à pH 7,4.

26. Procédé selon la revendication 24 ou 25 où les effets secondaires sont des effets secondaires de bradycardie, hypotension ou tachycardie.

27. Procédé pour identifier un agent thérapeutique potentiel qui comprend la mise en contact d'un composé test avec un récepteur d'adénosine dans un tissu, cellule ou membrane pathologique et un tissu, cellule ou membrane normal(e) correspondant(e) à une concentration inférieure à la valeur EC50 du composé test au récepteur d'adénosine à pH 7,4, et la détermination de ce qu'il y a une quelconque différence dans l'action du récepteur d'adénosine en réponse au contact avec le composé test entre le tissu normal et le tissu pathologique.

28. Procédé selon la revendication 27 où le composé test est identifié comme un agent thérapeutique potentiel si le composé test est un agoniste du récepteur d'adénosine, et l'action du récepteur d'adénosine en réponse au composé test est plus grande dans le tissu pathologique que dans le tissu normal.

29. Procédé selon la revendication 27 ou 28 où le tissu pathologique comprend un tissu épithélial.

30. Procédé selon la revendication 29 pour identifier un agent thérapeutique potentiel pour traiter le psoriasis, l'asthme ou la COPD.
